# EUROPEAN PATENT APPLICATION

(11) **EP 0 995 466 A1**
(43) Date of publication of application: **26.04.2000**
(21) Application number: 98910748.7
(22) Date of filing: 31.03.1998
(51) Int. Cl.: A63B 69/16, G09B 9/052

(54) **SIMULATOR PRECISION DEVICE APPLIED TO CYCLING**

(71) Applicant: Alvarez Fernandez, Manuel, 36210 Vigo (ES)
(72) Inventor: Alvarez Fernandez, Manuel, 36210 Vigo (ES)
(86) International application number: ES9800081
(87) International publication number: WO9949942

(57) **Abstract**

The device os the invention is specially intended to the training and improving the performances of and individual when using his or her own bicycle. The device is comprised of a special kinetic resistance electronic unit (31 and 32) which emulates with precision the dynamic effect of the mass of the user while controlling the resistance torque. A mechanical system of floating axles (21) ensures full operation of the bicycle. Electronic sensors situated at various locations send signs to the electronic control unit (31) which exchanges continuosly information with a computer or terminal (4) where in dynamic variables, physiological parameters and route profiles can be displayed and emtered in order to simulate a real competition situation and to make it more convenient for medical specialists to control the performances of the cyclist along the simulated traject. Special computer programs executed from the computer (4), enable to combine the presentation of the parameters and to generate historic or customised training programs which can be introduced or displayed locally or from a remote location by means of another computer with the use of an appropriate modem.

## Description

### OBJECT OF THE INVENTION

The present invention talks about to a simulator device of precision applied to the cycling for use in centers of high performance, gymnasiums, disciplinary centers, sport events of cycling or for domestic use. Specially useful when it is very important that the individual uses its own bicycle to make most similar tests of effort or programs of training to the reality of the highway or the track; when a pursuit combined and precise of the effort and the physiological parameters of the individual is required; when this pursuit has to be made simultaneously of one or several individuals, that are displaced of the pursuit center; when it is desired to have a file of its evolution throughout the sport season or is tried to correct defects in this activity that make difficult a good yield of the sportsman, as they can be: the inadequate use of its physiological characteristics, the irregular effort of both legs, an incorrect position on the bicycle or others.

The simulator device described here is of special application in the surroundings of the cycling of high competition, as much of highway as of track.

### ANTECEDENTS OF THE INVENTION

Although do not know antecedents which an equipment exists like which it characterizes this invention, since its fundamental application is focused to the sport of the bicycle, will be made a brief exhibition of some existing machines in the market commenting some of his characteristics, trying value better if it fits the features of the equipment of the invention.

In the market some and varied types of apparatuses used in the surroundings of the sport of the bicycle exist that are used to make tests of effort and maintenance; from the simple and well-known rollers, to trestle or frames specially developed for tests of effort of sportsmen in any level of competition.

With the rollers it is necessary to use a bicycle that is to work obligatorily On guard vertical, with the wheels supported on them; some equipped with a unit of resistance and a small steering wheel make a more comfortable pedaleo possible, but the obtained resistant pair is small because the power applied to the pedals is transmitted to the unit of resistance through the contact of the back wheel with the rollers; this produces certain slip, even though the transmitted power is not too high; asi same, does difficult to obtain precise data of the lost power in the rubbing when finding us with types of tubular covers or to different pressures of inflation in the used bicycle. On the other hand, the steering wheel only can emulate a small amount of mass very moved away of the real one. In any case, the utility of these equipment, thought fundamentally as a complement of the sport activity in times of bad weather, with the interesting characteristic to even use the own bicycle of the sportsman, is limited to make a maintenance of the muscular elasticity, to simply exercise certain agility in the pedaleo or to previous the muscular heating to a more intense exercise.

The trestle and frames thought for a sport activity more general than the rollers, have pedals, handlebars and a saddle that allow an exercise that resembles that it becomes in a bicycle, reason why also they are known with the generic name of static bicycles.

These apparatuses allow, in addition to the exercise, the control of the effort, for which they use different techniques that give a greater or smaller degree him of sophistication, finding us with equipment of very simple accomplishment composed solely of a small steering wheel and a unit from resistance with little exactitude, until equipment which they make a control of the quite acceptable effort, to the time which they provide information of other parameters and they facilitate the introduction and execution of programs that control the level of load based on certain time intervals.

Due to the different systems that exist in the market to provide a certain level of features, two of these systems will be analyzed that in any case contain the features of the others.

First of them, very used in gymnasiums by the facility of his handling, since from the sitting position on the saddle their parameters can be modified, one is made up - in addition to the elements that confer the characteristic to him of static bicycle - of an electronic system that is in charge of the visualization and control of parameters, an electric generator where the resistant pair takes place and a steering wheel that facilitates a more agile pedaleo. In a panel mounted on the handlebars one has access to the controls that allow the programming and visualization of the parameters related to the exercise that is going away to make and that can be: number of pedaladas, crossed space, speed, time of duration of the exercise, resistant pair, power, made work, number of calories supposedly consumed, pulsations, etc.

The unit of kinetic resistance formed by a electric generator controlled by the electronic system, not that allows a very acceptable control of the power loss, but the resistant pair, reason why upon the moment for making an exercise it agrees to maintain a pedaleo constant so that therefore a constant pair also stays. On the other hand, this power is limited by the size of the generator, that as well it is in favor it by the space available within the equipment and of the lack of an element able to dissipate the energy that in a while certain can be obtained from the exercise of a sportsman. For that reason, and with the purpose of protecting the system, the own electronic control is in charge to put limit to the resistant pair and the maximum power to dissipate that it is had to demand to the equipment. To this circumstance it is necessary to add the low effect of inertia, due to the low relation speed-mass of the steering wheel, whose effect is accentuated in the measurement that is increased the resistant pair; on the other hand, the disadvantage of not being able exists to make in this type of machine a good ergonomic adaptation of the sportsman, which is worrisome if we consider that the sportsman in his practices habitual, has developed a special musculature in a machine different from which is in this case.

We have then, a machine relatively either equipped for a control with the consumption of calories, with information sufficient to obtain an exercise methodical and or programmed to maintain a weight balanced at the times of noncompetition; probably very adapted for its use in gymnasiums, but moved away to cover the necessities with the professional cycling.

Finally, the second system, of almost identical construction to first, difference fundamentally in the procedure that it uses to obtain the resistant pair and in which it has a steering wheel of considerably greater inertia although with a mass far from the ideal.

The resistant pair is obtained by the rubbing of a tape of suitable material, that in band form surrounds the perimeter by a steel disc, that as well for of steering wheel of inertia and dissipator of energy. Tightening more or less the tape on the steering wheel a greater or smaller level of load is obtained, which is measured by a metallic arm that can turn freely on a one of its ends drawing up arc of 90 degrees and whose trajectory properly is calibrated. In order to obtain the influence of the arm on the load, this one is made rest on the tape through a support that is arranged to little distance of the spin axis; the other end of the arm incorporates a counterbalance calibrated whose push downwards produces a handle effect on the tape, of such way, that to the tightened being, produces a displacement in the arm that is mathematically function of the own effect of handle respect to the pressure of the tape on the disc and consequently of the asked for resistant pair. It can to say that the precision of the values presente in the calibrated scale is acceptable but not absolutely exact, had mainly to that the coefficient of friction of the tape on the surface of sliding of the disc is modified simply with the tip speed of the disc, with the temperature or with the relative humidity of the air, for this reason is also advisable to maintain a regular pedaleo at the moment for putting in practice an exercise.

This form to obtain pair resistant allows to obtain powers more high, than necessities cover with tests of effort with precision acceptable and with effect of inertia also improved, although not lack can to forget connection of cyclist with machine as it commented previously, which in this case it must more be watched, when allowing to make more effort and therefore to increase the risk of muscular injuries. However, the sport medicine specialists, for want of equipment able to provide better features, choose by this last one.

As complement to this exhibition is suggested to consult I articulate of Jose Manuel Castillo published in the sport newspaper Mark with date 08/02/1995 and titleholder "INDURAIN VERIFICA SU TURBO", where in different photos from Guillermo Rodriguez, he can see the champion of the world put under a test of effort by the specialistic sport medicine outstanding Drs. Sabino Padilla and Guillermo Cuesta, in a machine like which he finishes describing last.

### TECHNICAL PROBLEM TO SOLVE

The technical problem is focused on the one hand, to resolve that a single machine allows to a perfect connection between the sportsman and this one, to the time that is to be able to absorb the power that this one can simultaneously produce with its effort, obtaining to emulate the dynamic effect of the mass of each sportsman in individual and, naturally, that the precision of the measures cannot be influenced in any situation of work. On the other hand, like which one is treating in agreement with the invention is a simulator of precision applied to the cycling of high competition, this one has to be able to simulate a real situation of competition, (within its condition of static machine) and simultaneously to provide and with total exactitude the parameters derived from the exercise, that there are to serve the medical specialists to obtain the best yield of the sportsman in each one of his specialties. In addition, it has to allow the physical preinns (mainly at times of the Olympic Games) to follow from his centers of work the evolution of the sportsmen in his places of residence.

### CHARACTERIZATION OF THE INVENTION

The simulator device of precision applied to the cycling in agreement with the invention, is applicable in centers of high performance, gymnasiums, disciplinary centers, sport events of cycling or for domestic use and of a very special way to the cycling of high competition in anyone of its modalities.

It is characterized by his capacity to simulate a real situation of competition, creating the resistant pair in each passage of the route based on the passive slope and forces that take part, according to the speed that the same sportsman prints to the test, the type of highway or track, the anticipated wind, the type of wheel, etc.

It is characterized to emulate total precision the dynamic effect of the mass corresponding to the bicycle and the sportsman, even when this one loses weight as a result of a prolonged exercise.

It is characterized to use the particular bicycle of each individual and to allow that this one takes the slope gradient programmed to the time that allows all its operativity.

It is characterized to have a mechanical system of floating axles whose mission is to allow the connection and subjection of the bicycle to the simulator device with the same facility with which a wheel is replaced, to facilitate all the operativity of the bicycle and to transmit the energy applied in the pedals to the unit of kinetic resistance.

It is characterized by the use of a primary steering wheel, that makes the function of back wheel of the bicycle, allowing the performance of the back brake of this one to decelerate the march and serving as guide the transmitting strap; also it serves as pulley of friction to the tape hydraulic brake in the model of low features.

It is characterized to have an electronic unit of kinetic resistance whose mission is to create the resistant pair that is determined by the program that this executing itself and in addition, to accurately emulate the dynamic effect of the mass of the sportsman and its bicycle, considering that this mass changes in the sportsman at the most prolonged is the exercise that is making.

It is characterized by the capacity to communicate with other peripheral ones in local and remote way and to allow the introduction of routes obtained from real stages of competition, customized plans of work, combined visualization and synchronizes of own parameters and other equipment from other points of geography to a center of work, automatic generation of historical and a program opened to the incorporation of new advances.

Finally, it is characterized by its special functionality to provide different levels from features that allow to adapt it to a more general market; thus, they are obtained in addition to the equipment of general features, a model of average features and a model of low features.

### BRIEF STATEMENT OF THE FIGURES

One more a detailed exhibition of the invention can be come off the description of the same one on the basis of the following figures:
- figure 1 shows general an isometric view of the simulator device of mounted precision.
- figure 2 and figure 3 respectively show the isometric view of two variants of the simulator device with less features denominated of features average and low features.
- figure 4 and figure 5 respectively show to the Vista in plant and profile of the simulator device of precision.
- figure 6 shows the cut of a complete section of the mechanical system of floating axles.
- figure 7 and 8 presents the bicycle mounted on the simulator device in two different situations of work.

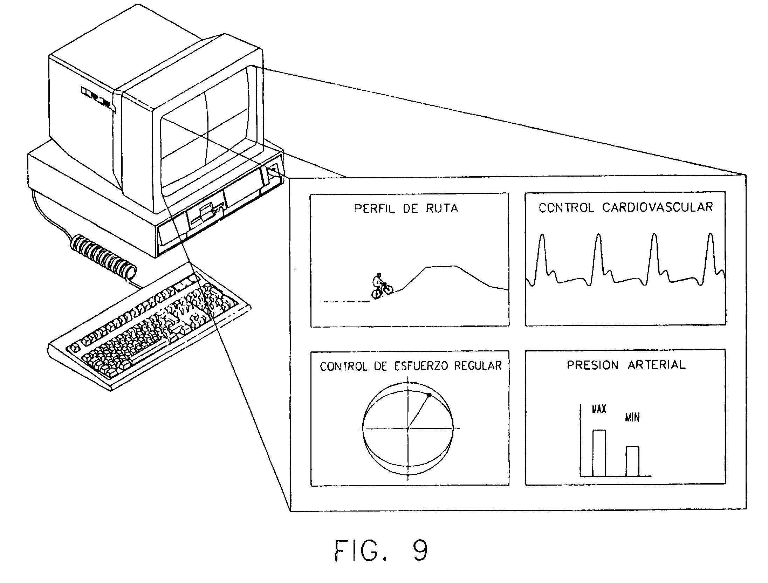

- figure 9 shows a screen of the computer where an example can be seen of as different parameters can be visualized simultaneously.
- figure 10 shows the title page of the terminal module of the variant of average features.
- figure 11 shows a synoptic scheme of the electronic unit of kinetic resistance.
- figure 12 shows the primary steering wheel with the detail of the tracks of braking and guides.

### EXHIBITION PRACTICES OF THE INVENTION

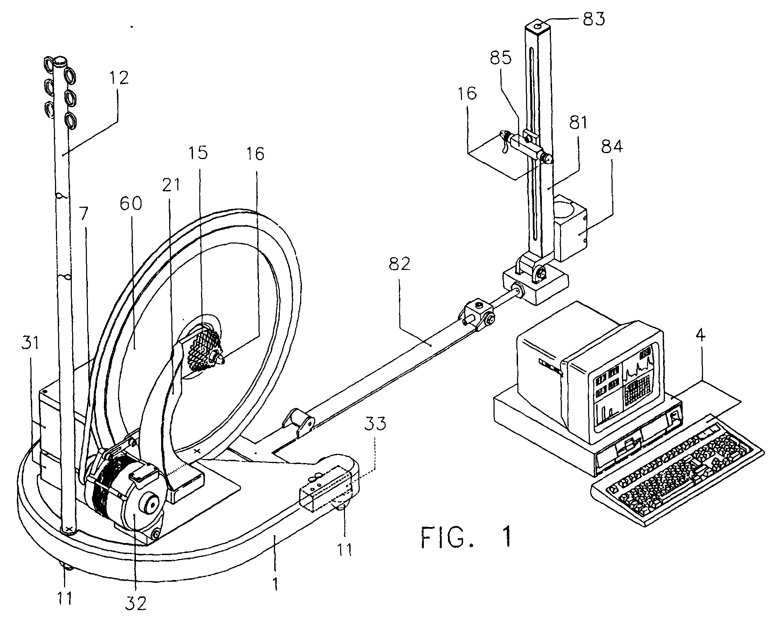

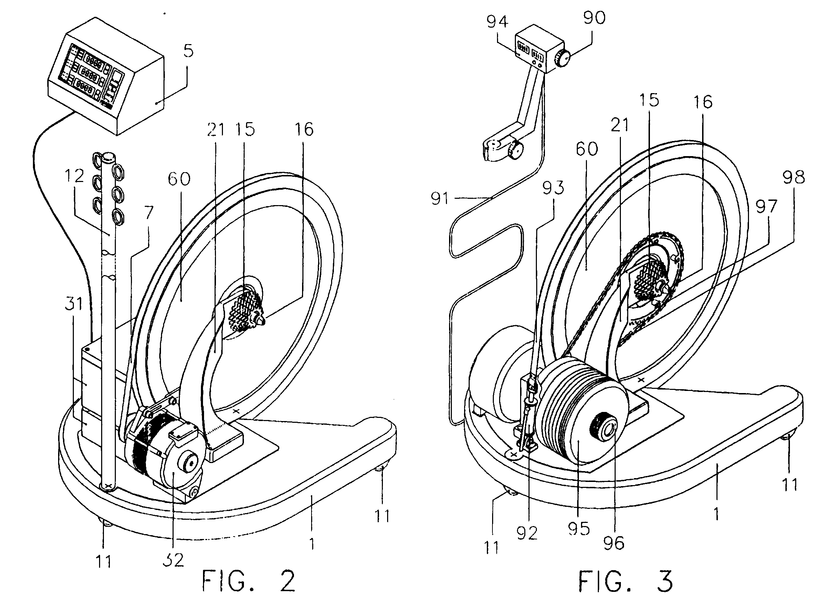

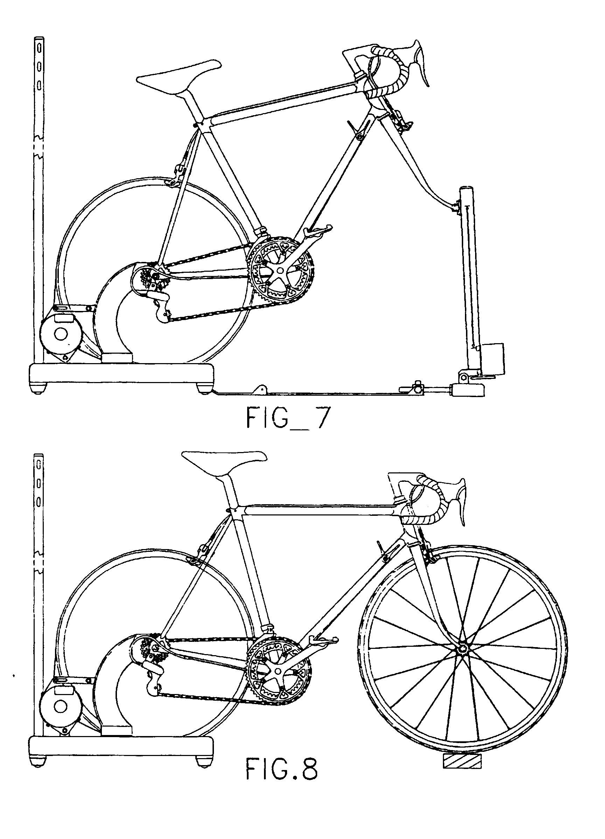

As already it commented previously, the simulator device of precision applied to the cycling (fig 1) is an apparatus able to simulate a real situation of competition, as much if it is highway like of track Therefore, the first necessity that is had to solve is the use of the bicycle of each individual in individual, that in addition has to have all its operativity. For it a special mechanical system of floating axles has been developed (21 to 25) that it is screwed to a base (1). Its mission is to allow to the connection and subjection of the bicycle to the simulator device, like can be seen in figures 7 and 8, with the same facility with which the back wheel is replaced, thanks to that it has the same fast system of blockade (16) that use the professional bicycles; it contains the game of interchangeable pinions (15) that is had to use in each test and a primary steering wheel (60) in form of biconvex wheel that contributes a small amount of mass to the time that serves as transmitting pulley of the energy applied in the pedals towards the electronic unit of kinetic resistance (31 and 32), where takes place the resistant pair and the effect of the mass is emulated. In the case of the model of low features (fig 3), a tape hydraulic brake (90 to 93) has the function of kinetic resistance yielding the energy in the same primary steering wheel (60) where it dissipates in heat form.

The characteristics of construction of this system of floating axles (21 to 25) allow to the bicycle all their operativity. Thus it is possible to obtain, at the same time that pedalea, that the bicycle takes the slope gradient (fig 7) and to manipulate all the speed changes or to restrain the march of the steering wheel; in addition, because the axles turn on bearings of balls (24 and 25), the lost ones by friction are extremely small and in any case easily controlable.

This mechanical system of floating axles (21 to 25) along with the electronic unit of kinetic resistance (31 and 32), that is described next, confers to the simulator device of precision their more remarkable and novel characteristic in the scope of the machines developed for the sport in general, but mainly in the cycling, since starting off of the specific function of these elements, we can simulate a real situation of competition and study at the same time throughout the route, the physiological evolution of the sportsman based on its effort and with such reason, to determine as it has to dose his effort to obtain an optimal yield of his.

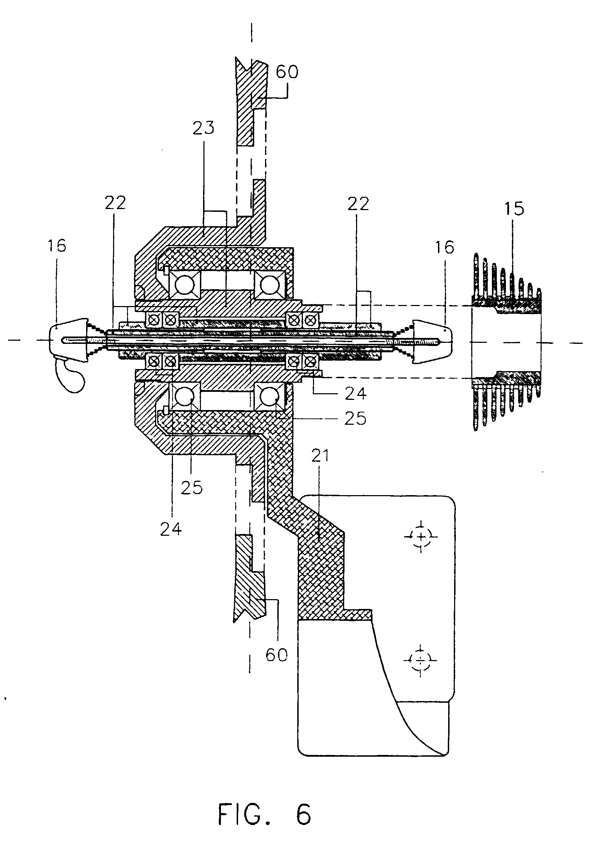

The mechanical system of floating axles (21 to 25) that it is possible to be seen sectioned in fig 6, is constituted by three bodies that form three concentric axles that can turn independent on a same center, these are: the main body (21), the floating body (23) and body of the primary axis (22).

The main body (21) serves as pedestal and support of all the assembly. In its interior the floating body (23) through two bearings leans (25) that allow a smooth sliding him with a high mechanical efficiency; he is subject to a base (1) and their constructive characteristic along with the one of the floating body (23) makes possible that the primary steering wheel (60) can work suspended, making the functions of back wheel of the bicycle, at the same time that allows the connection of this one to the simulator device with great comfort and guarantees the correct activity of the mechanism of change of pinions of the bicycle.

The body of the primary axis (22) has the function to hold to the bicycle by the back bracket in the same point where it makes his wheel back and allow at the same time that the bicycle can take the slope gradient that determines the program at every moment; it is supported in the floating body (23) through four bearings (24) that as in the previous case, they allow a smooth sliding with a high mechanical efficiency; a longitudinal drill practiced in the center of the axis and the special form of its ends, allows the use of the system of standard blocking fast (16) that use the professional bicycles, reason why the connection of the bicycle to the simulator system is executed in seconds.

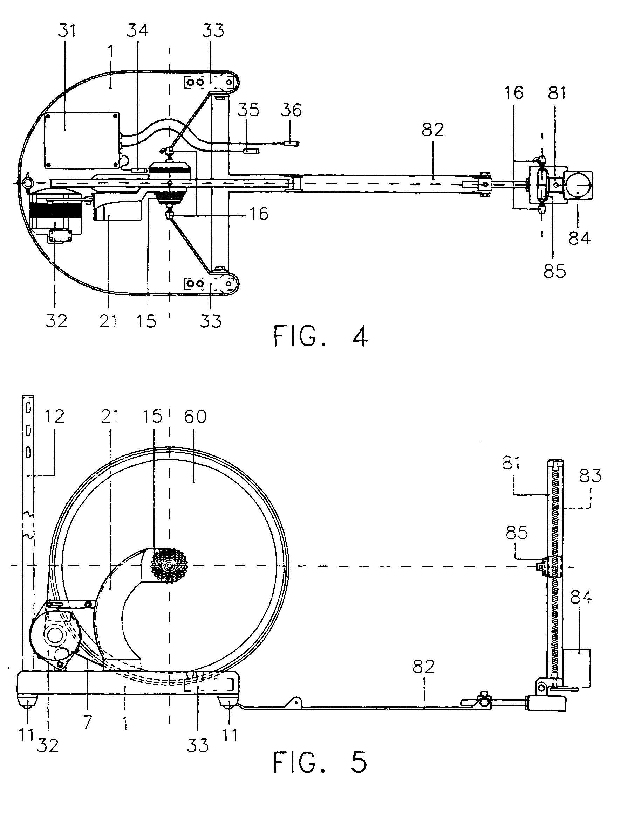

The floating body (23) is located between the main body (21) and the body of the primary axis (22), and while the main body (21) is subject to the base (1) and the body of the connected primary axis (22) to the bicycle (to see fig 4 the 8), the floating body (23) tour freely between both. Its mission is to transmit the energy applied in the pedals, cleanly and without influencing in no other element of the system, doing of mechanical transmission between the bicycle and the unit of kinetic resistance (31 and 32), happening through the primary steering wheel (60), and the transmitting strap (7) if the electronic system is used (fig 1 and fig 2), or to the primary steering wheel (60) directly if the tape hydraulic brake is used (90 to 93) (fig 2). To it, in one of his heads it has a spiral zone where the interchangeable game of pinions (15) chosen for the test, that lodges it is located exactly in the same levels and under the norms that regulate the measures correctly so that the changes of march are executed with cleaning and, thanks to this the use of games of commercial pinions is possible. The other end of the floating body (23) has a form specially adapted, to contain the primary steering wheel (60) with form of biconvex wheel and to along with obtain the main body, that this one perfectly works trim on the bracket replacing the back wheel of the bicycle; thus one is able to also give his own utility to the brake of the bicycle that can decelerate the march acting on a special track (61) that stops it arranges this primary steering wheel (60)

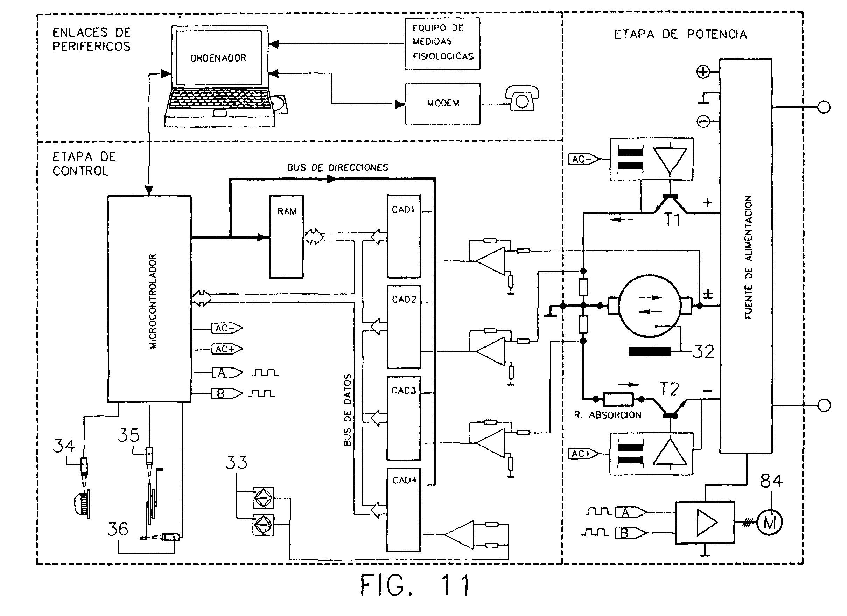

The electronic unit of kinetic resistance (31 and 32) has been developed to create the resistant pair that at every moment is asked for by the program that is being executed and to accurately emulate the dynamic effect of the mass corresponding to the sportsman and its bicycle, considering that this mass changes in the sportsman at the most prolonged is the exercise that is making. It is connected with the computer (4) or with the electronic terminal (7), through a port of communications, from where it receives information of the work program and it sends information of the dynamic variables as well that later suitably they are treated and visualized Also it receives information of the cells of load (33), detector of speed (34), detector of connecting rod (35) and detector of chain (36), so and as it is possible to be seen in fig 11.

The operation of the electronic unit of kinetic resistance (31 and 32) this based fundamentally on two elements, these is: a generator (31) that produces the dynamic variables on the primary steering wheel (60) that does the times of back wheel of the bicycle and, an electronics (32) that controls them and it is in charge to dissipate or to provide the energy necessary to emulate the dynamic effect of the mass. In the process of work of this unit two dynamic parameters are combined simultaneously. One is the resistant pair, that is the force that is had to win so that the bicycle stays in movement. It depends on the profile of route and the coefficients of other passive forces as type of firm, speed and wind direction, type of wheel, etc. if a test of competition or the load corresponding to the effort to is being simulated which is tried to put under the individual if it is a test of effort. Other parameter is influence of mass (as it is known, has to see with weight of sportsman and his bicycle) and his effects of inertia, that is the force that is against to that this mass changes of state, of such way, that if is tried to modify the speed of march, is had to also modify the applied force, that is based on the mass and of the time that takes in reaching the wished speed. Tying to make specific better the previous thing, it is possible to be said that the acceleration of the march will be equal a: acceleration = (applied force - even resistant) / mass. This one is the basic principle that defines the particular performance of the emulator of the dynamic effect of the mass within the electronic system. In order to obtain that all this is fulfilled, the generator (31) will make the function of generator, taking the kinetic energy from the pedals through the primary steering wheel (60) to transform it into electrical energy and to yield it to the base (1) in heat form through the transistor of power T2 and an absorption resistance when it is had to control the resistant pair, a positive acceleration or both. It will do of motor, taking energy from the electronic equipment through transistor T1, yielding it to the primary steering wheel (60) when it is had to control a negative acceleration if the pedaleo rate is reduced and, controlling a positive acceleration if it is simulating a slope downwards, as if a mass really existed and this one was the one that yielded energy

The electronic equipment (32), by means of a microcontroller and the aid of specialized electronics, is in charge to detect and to control in real time, any mini change of force applied in the pedals of the bicycle, measuring the currents that circulate through the generator, to respond to the situation immediately that is had to create at every moment; the transistors of power T1 and T2, make the control of the load and acceleration that are had to produce in the generator (32). It is necessary to make an observation to understand better the action of the resistant pair in the scheme of figure 11: transistor T2 cortocircuita exit of generator (32) based on acceleration positive and of pair resistant solicitd, but so that the current is induced that really creates the resistant pair, is necessary that the generator (32) is in movement with a speed that at least allows to compensate its own fall of internal voltage. As a result of this and so that the system begins to work effectively, it is forced to exceed a rate of determined pedaleo, which would be clearing effectiveness to the simulator device. In order to avoid this disadvantage and that the system begins to be effective from the rest position, the generator (32) is fed with a small amount of electrical energy that produces a current that compensates its fall of internal voltage and this way to facilitate the obtaining of the resistant pair from the rest position. Naturally this current will cause the tendency to turn to the primary steering wheel (60) in inverse sense, but the transistor T2 is in charge to block the circulation of current while the steering wheel is in absolute rest or if it tends to turn in opposite sense, avoiding so the primary steering wheel (60) backs down as a result of this additional energy.

The cells of load (33), the detector of revolutions (34), the detector of chain (35) and the detector of connecting rod (36), also send information to the electronic equipment that it uses it and it sends it to the computer (4), or to the terminal module (5) according to the cases.

The cells of load (33) measure the weight of the sportsman at any moment to consider the possible variation of the mass. After to be previously introduced the weight of the sportsman and its bicycle separately through the keyboard of the computer (4), it is possible to be begun the exercise, moment at which the simulator device begins to take a series from samples of the reading of the cells of load (33) throughout the trajectory of the connecting rod of the bicycle, which they serve to determine a proportional variable in each point of sample, that will be based on the weight of the sportsman, its spread and the size of its bicycle due to the geometric position of the cells of load (33) respect to the influence point of the forces according to the position to after periodic form, successive comparisons are made such in sample points and this way it is verified if modifications in the weight of the sportsman have taken place, to be able at any moment to update thus the dynamic effect of the mass emulated by the simulator device.

The detector of revolutions (34) detects the angular displacement of the primary steering wheel (60) and sends the information in form of impulses to the microcontroller who transforms it into his equivalent tangential one and that she comes to be 5 mm of route; whenever this happens, an interruption in the program of the microcontroller takes place to update the information of the speed parameters, acceleration, route and the correction of the slope if there were it.

The chain detector (35) serves to detect in what position of the sprocket is working the chain of the bicycle thus and, with the joint analysis of other physiological data of power and, to know the relation the change as pinions where better yield of the sportsman has been obtained.

The connecting rod detector (36) detects the passage of beginning of cycle of the connecting rod; at the moment that this happens, the microcontroller begins to count the number of impulses that the detector of revolutions until a new passage by the cycle beginning takes place, with this information sends divides the trajectory of the connecting rod in so many positions as impulses have been detected Known east data it is possible to be determined at every moment in what position of the route is the connecting rod; analyzing this parameter along with the force applied at the same moment, determines the curve of effort throughout the trajectory of the connecting rod; this information is used later in the program of the computer to know if the sportsman makes a round pedaleo (regular effort in both legs) or how to manage it to improve its yield.

A support positioner (81 to 85), to see 5 fig and fig 7, located in the front part of the simulator and screwed device the base (1) by a mooring frame (82), has the mission to hold the bicycle by the bracket, replacing to the front wheel. Its function is to cause that the bicycle takes the slope gradient corresponding to the profile from the highway; to it it has a small spindle (83), by which the support moves (85) that holds the bicycle; this small spindle, is dragged by a motor (84) that is governed by the electronic control (31), based on the received information of the computer (4). Thus the difficulty of the slope will go bound with the inclination that will take the bicycle doing this way the agreed exercise with the reality of the highway. For effort or track tests, the use of this element can be avoided using the same front wheel of the bicycle as it is possible to be seen in fig 8.

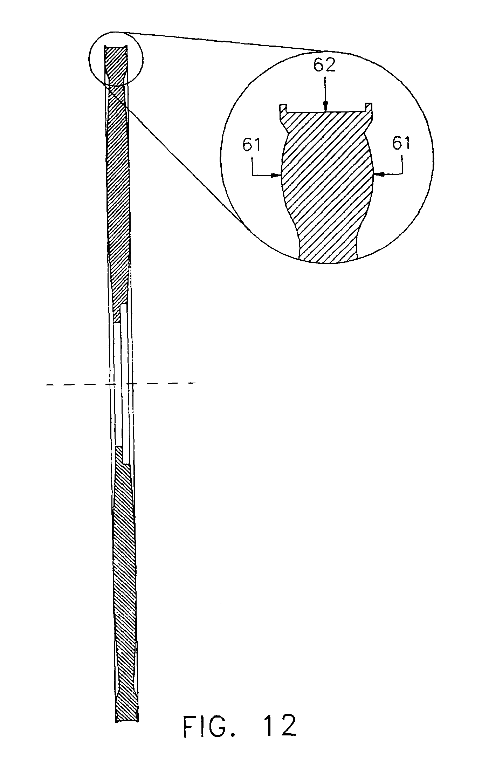

The primary steering wheel (60) has biconvex form (fig 12) and replaces the back wheel of the bicycle doing the function of front door and exit of energy towards inside and outside the bicycle; thus, when pedalea, the energy applied in the pedals is lead to the electronic unit of kinetic resistance (31 and 32) by the primary steering wheel (60) through transmitting strap 7 and, when it is let pedalear or the program is simulating a slope slope, it is also the primary steering wheel (60) that receives the energy of the electronic unit of kinetic resistance (31 and 32). If the model of low features is used (fig 3), the primary steering wheel (90) has an added function that is to serve at the same time as pulley of friction to the tape hydraulic brake (90 to 93) through the track of guide (62) and of dissipator of energy; also it receives the energy that in a while certain is yielded by the real mass constituted by the inertia discs (95) which they are arranged in this model of low features (fig 3). Another important function of the primary steering wheel (60) is to facilitate the action of the back brake of the bicycle to minorar the march when therefore it is required; to it it has two lateral tracks (61) located to the standard height of the brake shoes.

All the exposed one is of extraordinary importance for the cycling in any level of competition, since with this system the problem of adaptation between the sportsman and the machine is solved definitively, since it is using the hers own one. In addition, when taking the bicycle the slope gradient, the sportsman will feel stimulated to rise and to seat in the bicycle, which will make the exercise more pleasant and real. On the other hand, when emulating the dynamic effect of the mass of the sportsman is solved one of the most complex problems and important of any machine that there is to measure or to facilitate a sport activity, since this effect is different in each individual and to have controlled it also means that the effort with exactitude and in the same conditions is being moderate that actually real. However, we have to consider that when the sportsman exerts a force on the pedals makes also take part all its body; like consequence, they are created on the frame of the bicycle a assembly of disordered forces that give origin to a small amount of nonmeasurable energy by the simulator device. Naturally this situation also will occur in practices it real, reason why it will not have influence in which to the simulation function it talks about; nevertheless, it could be introduced in the total calculation whenever it is measured with another incorporated equipment and to the computer (4) through the table of coefficients

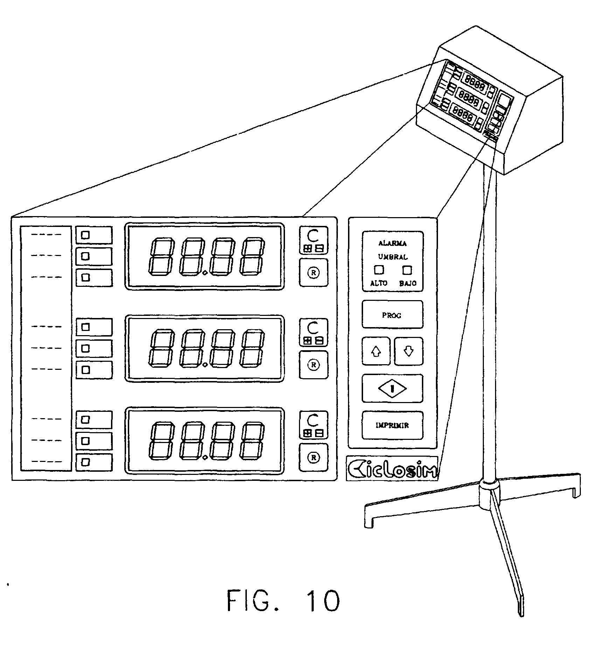

The use of a personal computer (4) confers to the device a great capacity to communicate locally or distances with other peripheral ones and also it leaves a laid way to the application of new advances. It incorporates special a computer science program, that allows the following functions: the introduction of the profile of a real stage, that previously has been taken with another equipment in the own highway and later transferred to the computer either through this equipment or from a support of data; the introduction of customized plans of training directly from the keyboard of the own computer (4), with the coefficients of the passive forces that take part in the exercise of a competition and that are added to the active work that is to make the sportsman; the connection with other medical equipment that have standard protocol of communications, to the obtaining and simultaneous graphical and numerical presentation of the dynamic variables derived from the exercise and the physiological parameters from the sportsman (to see fig 10); the introduction of the personal data of the sportsman and the generation of historical of each made test, that they can be maintained or be transferred to another computer if it is desired; the connection with other computers via modem to allow to the control and the manipulation of the work programs from a center of common work, connected to the different places from residence of the sportsmen and finally, the possibility of incorporating multimedia programs.

Finally, with the aim to provide different levels from features that allow to adapt it to a more general market, two variants are obtained that maintain the features most remarkable of the precision simulator but their general features diminish. In fig 2 and 3 these two variants can be seen in which no of the two uses the support positioner (81 to 85) that it allows that the bicycle takes the slope gradient; in fig 8 the bicycle supported on its front wheel can be seen.

The represented simulator device in the fig 2, that is denominated of average features, replaces the computer (4) by a terminal module (5), but follows the same philosophy explained previously to obtain the resistant pair and the emulation of the dynamic effect of the mass, unless it does not have cells of load (33) and therefore it cannot by itself update the mass of the sportsman if this one loses weight as a result of the exercise. It either does not have memory sufficient to store the profile of a real stage, but it allows to the introduction of customized plans of training and the simultaneous presentation of three parameters between a total of 15, physiological physicists and.

The represented simulator device in fig 3, maintains the functionality that provides the use to him of the mechanical system of floating axles but it changes the way to obtain the resistant pair and the emulation of the dynamic effect of the mass; it is denominated of low features. In order to obtain the resistant pair, it uses a system of tape hydraulic brake (90 to 93), made up of a piston or pump (90) that is united by a flexible pipe (91) to a cylinder of return by wharf (92) that throws of a brake lining (93) that surrounds to the primary steering wheel (60) friccionando on a track (62) specially prepared so that it can yield the energy in heat form. The operation is very simple: turning the button of control of the pump (90) towards a sense, a pressure in the hydraulic circuit is created that forces the cylinder (92) to move throwing of the tape (93) that surrounds to the primary steering wheel (60) embracing the track (62) and causing a friction on this one, that will be greater whatever more pressure exists in the also greater circuit and therefore the resistant pair; turning the control in inverse sense, the pressure vanishes relaxing the shot of the tape (93) and therefore the friction, until the primary steering wheel (60) is released completely leaving practically null the pair resistant. In order to determine the value of the resistant pair that is being applied at every moment, a transducer of pressure has been had located in the module control (94) that linealiza the measurement and directly presents it through a digital indicator in Nm or equivalent values of slope difficulty if it is desired; the measurement of the resistant pair thus obtained has a precision acceptable, but inferior to the one of the methods previously commented. The same module of commented control (94) previously contains a visualizer of standard type that is in charge to visualize instantaneous speed, average speed, distance, time, etc.

In order to emulate the dynamic effect of the mass, discs of inertia (95) that are calibrated in different thicknesses and engravings with the information corresponding to the mass are used that emulate, to be able to conform the total mass corresponding to the sportsman and its bicycle. These discs are dragged by the primary steering wheel (60) through a conductive pinion (97) and one transmitting chain (98). A nut of blockade (96) of round and knurled form, located in the end of the axis that it supports to inertia discs (95), serves to fix and to allow the change of these with facility.

The curve-slide supports (11), serve to facilitate the displacement of the simulator device and the mast (12) for support of the physiological soundings of the medical equipment if they are used.

## Claims

1. **PRECISION SIMULATOR MECHANISM APPLIED TO CYCLING** specially developed for training and imcreasement of the sport performance on the particular bicycle of each person. It has it's application in high performance rehabilitation centers, gymnasiums, domestic use and sport ciclist events, and it's main characteristic is to simulate a real life practice situation of competition either if it is carried out on route or indoors, due to a mechanical system of floating axles (21 to 25) which makes easier the whole bicycle operation; to an electronic system of kinetics resistance (31 and 32) which create the resistance couple and emulates the dynamic effect of the bicycle and sportsman mass; to a positioned support (81 to 85) which positions the bicycle with the required slope angle, to a primary steering wheel (60) which makes the back wheel function of the bicycle; and last, to a personal computer (4) in permanent communication with the electronic unit of kinetic resistance, which contains a special program where the route profiles and working plans are introduced, the parameters arising from the exercise are shown and the communication with other local or distant equipment is easier.

2. **MECHANISM AS PER VINDICATION** 1, distinguished because of the mechanical system of floating axles (21 to 25) has three parts (21, 22 and 23) which form three concentric axles resting on ball-bearings (24 and 25) which due to it's particular construction can rotate completely free on a same centre without interferences among them, so that each part can make its own function with a high mechanical performance.

3. **MECHANISM AS PER VINDICATION 2**, distinguished because of the mechanical system of floating axles (21 to 25) has main part (21) attached to the base (1), which holds up the primary steering wheel (60) through the floating part (23) so that the steering wheel (60) works suspended and at the same adecuate height in order to turn freely, guaranteeing at same time the correct function of the mechanism of the bicycle pinion change.

4. **MECHANISM AS PER VINDICATION 2**, distinguished because of the mechanical system of floating axles (21 to 25) has part of primary axles (22) leaneing on the floating part (23) through four ball-bearings (24) which connects the bicycle to the simulator mechanism, using the standard system of quick gripping (16) which is used in the commercial bicycles and which makes it easier at the same time for the bicycle to take the aclimbing angle foreseen in the exercise.

5. **MECHANISM AS PER VINDICATION 2**, distinguished because of the mechanical system of floating axles (21 to 25) has a floating part (23) that rests inside the main part (21), turns freely between this and the primary axle part (22) to transfer to the primary steering wheel (60) the energy applied to the pedals; has at one of it's ends a threaded part to lodge a set of pinions (15) and locates together with the principal part, to the primary steering wheel (60) centred in the back pitchfork of the.

6. **MECHANISM AS PER VINDICATION 1**, distinguished because the electronic system of kinetic resistance (31 and 32), is provided of an electronic unit (31) composed of a power card which controls one motorgenerator (32) and includes an additional feeding which makes possible that the resistance couple can be carried out from a resting position, and od a control card which contains a microcontroller which the variables that take part in the motorgenerator function and that is communicates to a computer (4) or with a terminal (5) in order to receive and send information about the dynamics variables which takes part on the exercise performance.

7. **MECHANISM AS PER VINDICATION 6**, distinguished because the electronic system of kinetic resistance (31 and 32), controls the sportsman loose of weight due to the exercise, because to the combined action of the electronic unit (31) and the motorgenerator (32); is added to the periodic information received from the charge cells (33) which controls the sportsman weight through all the connecting rod trajectory of the bicycle.

8. **MECHANISM AS PER VINDICATION 6**, distinguished because the motorgenerator (32) makes double function, one as an electric motor providing energy or such as generator requesting it, for obtaining in this way the dynamic variables which influence on the primary steering wheel (60) to produce the resistant couple and to emulate the dynamic effect of the mass.

9. **MECHANISM AS PER VINDICATION 1**, distinguished because the personal computer (4) contains a special computerized program which includes following items: it allows the introduction of a real lap profile, which has been taken previously with another equipment on the real road, and later on transferred to the computer either through this equipment or from a database; the training personalized plans introduced directly from the keyboard of the computer, with the passive forces coefficients which takes part in the exercise of a competition, and that are added to the active work which sportsman must make, the connection with other medical equipment which have a standard communications protocol (fig 11), for the obtention and simultaneous graphical and numerical presentation of the dynamic variables derived from the exercise and the sportsman's physiological parameters (fig 10);. the introduction of sportsmen personal data and the generation of historical of each trial carried out, which can be kept and transferred to another computer if desired; the connection to other computers via modem (fig 11) to permit the control and the manipulation of the work programs from a common place of work, to different places of residence of the sportsmen, and finally, the possibility of incorporating futures advances.

10. **MECHANISM AS PER VINDICATION 1**, distinguished because it has a chain detector (35) which allows to detect the chain position on the bicycle sprocket and this way together with the potency and physiological data,we get the relation of the bicycle pinions change where the sportsman gets a better performance.

11. **MECHANISM AS PER VINDICATION 1**, distinguished because it has a connecting rod detector (36) which together with the revolution detector (34), is known through the computer (4) the force bend along the rod detector trajectory, that allows to know if the sportsman is making a round pedal (same effort in both legs) or how to do it in order to improve his performance.

12. **MECHANISM AS PER VINDICATION 1**, distinguished because the primary steering wheel (60) has a breaking track (61) in each side, with which is possible the performance of the back brake shoes in order to be able to disaccelerate the steering wheel speed when the exercise need it, and also because it has a guide and friction track (62) with which the primary steering wheel action (60) to serve as a friction fan belt to the hydraulic brake (90 to 93] in the lower performances module (fig 3).

13. **MECHANISM AS PER VINDICATION 1**, distinguished because it can be transformed into a medium performances model changing the computer (4) by an electronic terminal (5) and removing the positioned support (81 to 85), or into a low performances modelo if the the electronic terminal (5) is also changed by a control module (94) and the electronic unit of kinetic resistance by an hydraulic lining brake (90 to 93) and by an inertia disc set (95).

14. **MECHANISM AS PER VINDICATION 13**, distinguished because the hydraulic lining brake (90 to 93) is composed of a piston or pump (90) which is joined by a flexible pipe (91) to a cylinder of return by spring (92) which pulls of the brake line (93) which surrounds to the primary steering wheel (60) frictioning on a track (62) making a mechanic unit of kinetic resistance with which the resistant couple can be obteined in the low performance module.
